# EUROPEAN PATENT APPLICATION

(11) **EP 2 664 293 A1**
(43) Date of publication of application: **20.11.2013**
(21) Application number: 12168524.2
(22) Date of filing: 18.05.2012
(51) Int. Cl.: A61B 18/12, A61B 18/14

(54) **Interactive electrophysiology catheter**

(71) Applicant: Wimed I/S, 2720 Vanløse (DK)
(72) Inventor: Matthiesen, Mads Emil, København N DK-2200 (DK); Paamand, Rune Tore, Vanløse DK-2720 (DK); Shah, Manish Hasmukh, Washington, DC District of Columbia 20010 (US)
(74) Representative: Kuhn, Oliver Wolfgang

(57) **Abstract**

Problems in the CathLab are centered on lack of control to the physician. Most control is achieved in the remote work solution. Solution: A catheter assembly comprising a user interaction system provided at the handle, the user interaction system comprising at least one user input sensor enabling the user to provide input to the catheter assembly, the user interaction system comprising at least one output generator for providing output to the user.

## Description

The present invention relates to minimally invasive catheters used for electrophysiology measurements to diagnose heart arrhythmias.

Minimally invasive catheters employed to diagnose heart arrhythmias are well known. They are used to facilitate the creation of electrograms from electrophysiology (EP) signals measured inside the heart (intracardiac) by electrodes associated with such catheter. Diagnostic catheters are well known and comprise a flexible tube attached to a handle. When connected to electrophysiology mapping systems today, cables conduct analog signals away from catheter's handle to other processing instrumentation. The cable's other end is typically split into a plurality of cables corresponding to the number of electrodes. EP labs are interchangably called CathLabs.

It is an object of the present invention to provide a catheter assembly having an improved usability.

According to a first aspect of the present invention, there is provided a catheter assembly comprising an elongated member, a plurality of electrodes, a handle, and a user interaction system. The elongated member may have a proximal portion and a distal portion, the distal portion being configured to be inserted into a mammal body. The plurality of electrodes may be provided at the distal portion of the elongated member. The plurality of electrodes may be configured to provide one or more input signals in response to electrophysiology signals being present at the electrodes. The handle may be connected to the proximal portion of the elongated member. The handle may enable a user of the catheter assembly to maneuver the elongated member during insertion of the distal portion within a mammal body. The user interaction system may be provided at the handle. The user interaction system may comprise at least one user input sensor enabling the user to provide input to the catheter assembly. The user interaction system may comprise at least one user output generator for providing output to the user.

According to a second aspect of the present invention there is provided a method for electrophysiology measurements within a mammal body. The method comprises utilizing the catheter assembly according to the present invention for obtaining electrophysiology measurements.

EP systems typically comprise catheters, data acquisition systems, work stations and monitors with a subset of other instrumentation such as junction boxes, amplifiers and patient interface units. Measuring key values on the electrogram is typically performed at a more remote work station. According to the present invention, an EP system is simplified to only comprise catheters and monitors, with an optional compact control unit in between.

Diagnostic catheters may perform additional functions other than sensing. Catheter maneuvering is important to reach the right location in the heart, and mechanical arrangements are sometimes employed to bend the tube's distal portion. Diagnostic catheters are also known to deliver electric currents at regulated variables, referenced as 'pacing' or 'stimulation'. Catheter electrodes are sometimes utilized to measure impedance and thus estimate tissue contact. Catheters can include a pressure sensor at its distal tip to estimate risk of tissue perforation. According to embodiments of the present invention, a catheter interacts with the physician to assist configuration. Configuration is typically pairing channels, setting pacing, setting monitor display, steering the flexible tube and setting electrodes for impedance measurements. According to the present invention, the catheter interacts through feedback on configuration or biophysical measurements, such as electrode sensor signals, pressure sensor signals or impedance measurements.

According to the present invention, a sensing catheter may be interactive. An interactive catheter may allow for feedback on biophysical measurements (i.e. e.g. input signal(s) provided by at least one of the plurality of electrodes), such as cardiac electrograms, impedance measurements or pressure measurements. The catheter may output such measurements through vibration, light and/or sound. According to the present invention, the interaction may include user input interfaces such as button or trackball for finger control and/or microphone for voice control. The present invention may provide means for one or more of the following: pacing, preventing tissue perforation, maintaining tissue contact and measuring electrograms.

Referring back to a first aspect of the present invention, a user is empowered by direct interaction with a catheter.

An interactive catheter as described in the present invention promotes improved and intuitive workflow for the user. As such, the user is enabled to utilize the catheter as an interactive tool in configuration and measurements during the procedure. This will eliminate misunderstanding between personal in the operation room and control room. It will save time walking between patient and computers. The catheter feedback can further assist the insertion and maneuvering of the catheter. This will save bundles of wires, troubles with worn out pins, and safety associated with hygiene when removing and re-attaching cables after rotating the handle.

A method for electrophysiology measurements within a human body with a interactive catheter assembly is described. The method implies a physician being present at the patient bed throughout the entire procedure, saving time, cost and improving control, hygiene and safety.

### BRIEF DESCRIPTION OF DRAWINGS

FIG. 1 illustrates an embodiment of a catheter, wherein the user input sensor and output generator are depicted;
FIG. 2 schematically illustrates an embodiment of a catheter, wherein the stimulation control and circuitry for signal processing is schematically depicted;
FIG. 3 schematically illustrates an embodiment of a catheter, wherein the vibration of the handle is shown;
FIG. 4 schematically illustrates an embodiment of a catheter, wherein the impedance measurement is depicted;
FIG. 5 schematically illustrates an embodiment of a catheter, wherein the voice recognition is shown;
FIG. 6 schematically illustrates an embodiment of a catheter, wherein the motor invoked movement is depicted;
FIG. 7 illustrates an embodiment of a catheter, wherein the motor associated mechanics is shown;
FIG. 8 schematically illustrates an embodiment of a system, wherein the catheter communicates with the EP system and interacts with a user through input/output generators;
FIG. 9 schematically illustrates an embodiment of a system, wherein a plurality of catheters communicates with the EP system through a catheter control unit and interacts with the user through input/output generators.
FIG. 10 illustrates an embodiment of a system, wherein 3 catheters communicates with 1 monitor.
FIG. 11 illustrates an embodiment of a system, wherein 2 catheters communicate with 1 catheter interface unit that further communicates with 2 monitors.

Like reference symbols in the various drawings indicate like elements.

### DETAILED DESCRIPTION OF EMBODIMENTS

The at least one user input sensor may comprise a microphone facilitating voice recognition. Voice recognition may involve a microphone for voice recording and software comprising digital signal processing for classification of recorded signals. The digital signal processing may further comprise methods such as using predefined signal sequences as templates for recognition of new recorded signals and/or frequency-based analysis. Voice recognition may be used together with other members of the user interaction system. In one implementation the user may use another user input sensor to confirm inputs from voice recognition. An example of such an implementation is a feedback loop where an outcome from the voice recognition software is outputted to the used through a speaker or display but no action is initiated until the outcome is confirmed by the user through use of another user input sensor. Voice commands may comprise measuring intervals on electrograms, setting pacing parameters and changing monitor view. An user input sensor on the catheter handle incorporating voice recognition will reduce misunderstanding and time expenditure communicating between an operation room and control room

The at least one user input sensor may comprise a finger controllable part, such as a touch and/or force sensing element. For example, and among others, the user input sensor may be a push button, a trackball, an optical trackpad, a touch screen or panel, a proximity sensor, an impedance sensor or an capacitance sensor. The user input sensor may be operable by one or more fingers. A finger controllable user input sensor will allow for immediate adjustments at the patient bed.

The at least one user output generator may comprise a vibration generating element. The vibration generating element may comprise a rotating element with a center of gravity different from the axis of rotation. The vibration generating element may be configured to produce vibration at different intensities. For example, the intensity may be varied in response to biophysical measurements, such as electrode sensor signals, pressure sensor signals or impedance measurements. The vibration generating element may be a member of a feedback loop. The vibration generating element may provide output to the user in response to configuration of the catheter assembly and/or the EP system. Vibration signal in response to pressure measurements from the tip of the catheter will be valuable for the physician to safely maneuver the catheter assembly.

The at least one user output generator may comprise a display. The display may provide visual information to the user. The display may comprise any display technology such as LED, LCD or other. The display may feature one or more display colors. The display surface may be planar or curved. The display may comprise light to allow different levels of illumination. The level of illumination may be configurable and may be automatically adjusted in response to circumstances such as ambient light or configuration of the catheter assembly. The display may be a member of a feedback loop. The display may provide output to the user in response to configuration of the catheter assembly and/or the EP system. Display output showing pacing settings to the physician will improve safety.

The at least one user output generator may comprise a speaker. The speaker may be configured by the user, the catheter assembly or other members of the EP system. The speaker may be a member of a feedback loop. The speaker may provide output to the user in response to configuration of the catheter assembly and/or the EP system.

The catheter assembly may be configured for providing output to the user in response to input signals. The output may be provided by the at least one output generator. The input signals may be provided by the electrodes or their simple sensors disposed on the elongated member. As such, the input signals may be provided by biophysical measurements, such as electrode sensor signals, pressure sensor signals or impedance measurements. The input signals may be provided by other members of the EP system. The output and input signals may form a feedback loop. The user may use the feedback loop for configuration of the catheter assembly.

The distal portion of the elongated member of the catheter assembly may be bendable and the catheter assembly may comprise means for controlling the bend of said distal portion. Said means for controlling the bend may further comprise motorized catheter steering. Said means for controlling the bend may be controlled by the at least one user input sensor or biophysical measurement. Said means for controlling the bend may be configured in a feedback loop. A feedback loop may improve the performance of the catheter assembly by automatic adjustment of the bend of the distal portion of the elongated member of the catheter assembly. In one implementation, the impedance of one or more electrodes are a function of the bend of the distal portion of the elongated member and is regulated by means for controlling the bend of said distal portion.

The system for electrophysiology measurements within a human body may comprise the catheter assembly and electrophysiology system. An electrophysiology system is a term used for the whole ensemble from catheters to monitors in a CathLab. An electrophysiology system may comprise at least one monitor and a data acquisition system. Electrophysiology systems may further comprise an amplifier, a junction box and other analog and digital instrumentation. Electrophysiology systems are interchangably called electrophysiology mapping systems. The catheter assembly associated with the electrophysiology system allows the user to control any aspect of electrophysiology measurements. As an interactive catheter assembly, the electrophysiology system may pass information to the catheter assembly that is then confirmed directly through the handle. As an interactive catheter assembly, inputs through the handle may be passed on to the electrophysiology system where they are returned or confirmed.

The electrophysiology system may comprise a catheter control unit. Said catheter control unit may comprise a pulse generator configured for pacing of heart tissue. Pacing may be done via one or more of the plurality of electrodes associated with one or more catheters. Pacing may be defined as electrical stimulation of heart muscle cells. Pacing from a local catheter control unit is an important step away from the remote control room.

The at least one user input sensor may be configured for controlling the electrophysiology system. In one implementation, control of the electrophysiology system may include configuration of the catheter assembly, one or more monitors, the catheter control unit or other members of the electrophysiology system. In such an implementation, configuration may involve setting parameters related to pacing, control of biophysical measurement or display of electrograms. In one implementation controlling the electrophysiology system empowers the user to make measurements on recorded electrograms. In one implementation, controlling the electrophysiology system allows pairing of the catheter assembly with the electrophysiology system.

The electrophysiology system may be configured to provide output to the user via at least one of the at least one user output generators, the output from the electrophysiology system for instance being provided in response to input sensed via at least one user input sensor or via at leas tone of the plurality of electrodes.

The catheter assembly may be configured for providing information to the electrophysiology system information about past events related to the catheter. Past events may include information related to manufacturing of any member of the catheter assembly. Past events may, as an example and among others, comprise information of usage history, information from interaction with the catheter assembly, information related to configuration of the catheter assembly or information related to sterilization. The catheter assembly storing information about itself will allow for an intelligent catheter. Physicians would often like to know usage hours and recycling times to judge its performance.

The catheter assembly or the system of catheter assembly and electrophysiology system may be utilized for electrophysiology measurements within a mammal body. The method for utilizing the catheter assembly may comprise enhanced usability, more control, increased freedom of movement, higher hygiene and less interruption, among other factors.

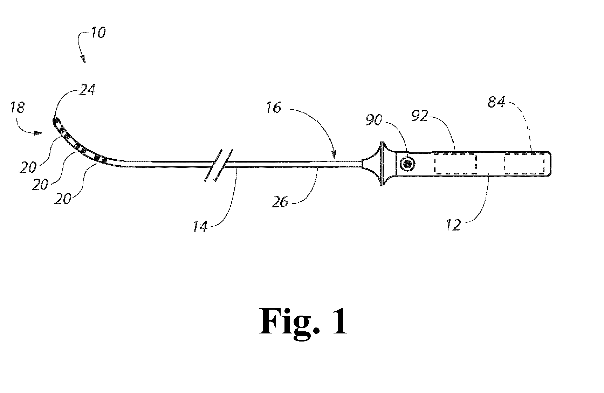

Referring to FIG. 1, an embodiment of a catheter is shown at 10. Catheter 10 includes an elongated member 14 and a handle 12 connected to the proximal portion of elongated member 14. In an implementation, handle 12 enables the user to maneuver the elongated member. In an implementation and as illustrated, elongated member 14 may include a proximal portion 16 and a distal portion 18. As depicted, a first arrangement of a plurality of electrodes 20 may be arranged on or around distal portion 18. In an implementation, the plurality of electrodes are configured to provide one or more input signals in response to electrophysiology signals being present the electrodes 20. Handle 12 includes a user input sensor 90 for user interaction. In an embodiment, the user input sensor 90 comprises a finger controllable part, such as a touch and/or force sensing element, such as a push button or/a touch panel. In another implementation, user input sensor 90 is trackball, an optical trackpad, a touch screen, a proximity sensor, an impedance sensor or an capacitance sensor. In an embodiment, a trackball facilitates detection of movement in two dimensions, can be activated as a push-button and may comprise multi-colored light indication. In an embodiment, input sensor 90 may be a flat optical trackpad to allow detection of movement in two dimensions and can be activated as a push-button. In an embodiment, input sensor 90 may further include multiple regions, each of which may provide independent interface means. For example, interface 90 may comprises two regions comprising push buttons, each of which may be independently pushed. Handle 12 includes an output generators 92 for providing output to the user. In an embodiment, the output generator 92 comprises a display. In another embodiment, the output generator 92 comprises a vibration generating element. In another embodiment, the output generator 92 comprises one or more speakers for generating sound. In an implementation, input sensor 90 and output generator 92 is an integrated unit capable of sensing input and generating output such as a touch sensitive display or a combined push-button and LED.

In an implementation, user input sensor 90 comprises a microphone facilitating voice recognition. In an embodiment, user input sensor 90 provides voice control that can be supplemented by other interaction such as trackball or buttons for control. With regard to both examples, the voice control and supplemented interactions may control parameters that are related to monitor display, measurements, catheter or channel selection, recording, saving data or adding comments or notifications to recording.

Handle 12 includes a module for communication 84. In an embodiment, the module for communication 84 comprises an isolation barrier. In another embodiment, the module for communication 84 further comprises means for connecting the handle 12 to a power source. In another embodiment, the module for communication 84 comprises wireless communication.

With continued reference to FIG. 1, in an embodiment, a biophysical sensor 24 is exposed on a tip of the distal portion. For example, and among others, the biophysical sensor 24 may be a pressure sensor.

With continued reference to FIG. 1, a second arrangement of one or more electrodes 22 may be positioned along elongated member 14. As shown, electrode 22 may be located at a position off-set from the first arrangement of one or more electrodes 20.

In an implementation, proximal portion 16 of elongated member 14 is generally straight and distal portion 18 is generally curved. In an embodiment, an electrically insulated conductor 26 extends from handle 12 longitudinally to distal portion 18 of elongated member 14.

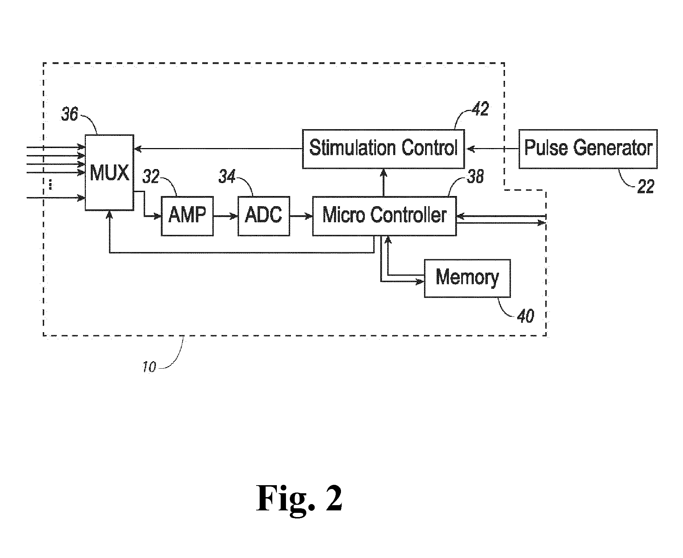

In an embodiment, interaction through input sensor 90 facilitates measurement of electrograms and control of pacing, for example, with a trackball that may be located on handle 12. In an implementation, handle 12 contains integrated circuitry for pacing muscle tissue and circuitry for processing sensor signals, each as shown in FIG. 2.

In an embodiment, a removable power source such as an adaptor is connected to the handle 12.

In an implementation, the input sensor 90 is accompanied by the output generator 92. In an implementation, the output generator 92 is a display configured to display parameters relevant to the use of the input sensor. In an embodiment the display may show a number proportional to the current curvature of elongated member. In an embodiment the display may show information related to pacing. In an embodiment the display may show information related to configuration of the catheter.

With reference now to FIG. 2, the circuitry for processing sensor signals include amplifying means 32, digitizing means 34, a multiplexer 36 and a memory module 40, and the circuitry for controlling electrical stimulation may include a stimulation control 42, both circuitries controlled and timed by a microcontroller 38. In an embodiment, a pulse generator 30 separate from the handle's circuitry delivers current to the stimulation control 42. In another, the pulse generator 30 is positioned inside the handle 12. In an implementation, microcontroller 38 relays incoming sensor signals and outgoing pulses; as is to be appreciated based on this disclosure, microcontroller 38 may control the separate components and processes inputs and may communicate information with data monitoring software (not shown) and memory module 40. In an embodiment, multiplexer 36, along with other components, may be controlled by microcontroller 38 and relays signals from stimulation control 42 to an output electrode 44 arranged on distal portion 18 of handle 12. In another embodiment, output from stimulation control 42 may be selectively controlled and regulated by an input sensor 90.

In an embodiment microcontroller 38 comprises a ceramic oscillator to control timing of the microcontroller and other components. In an embodiment, memory module 40 selectively stores desired events from microcontroller and other items of information communicated between microcontroller 38 and memory module 40. In an embodiment, memory module 40 may selectively store desired information experienced or generated by catheter, such as information related to pacing such as settings or limits for pacing parameters and the like.

In an embodiment, pulse generator 42 may comprise a trigger, timer and memory component. As will become appreciated based on the teachings of this disclosure, the trigger may comprise a input sensor such as push buttons located on the catheter handle 12.

In an embodiment, output from pulse generator 42 is connected to an indicator that may be located on handle 12. Among other examples, indicator may be a LED, but it is to be appreciated that the indicator should not be limited to an LED.

In an embodiment, the timer initiates the generation of a pacing pulse as a function of sensor signals. Sensor signals may be electrode measured muscle activity. In an embodiment, the catheter is programmed to initiate pacing if heart rate plummets under a predetermined value.

The amplitude of the generated pulse may be varied as a function of measured cardiac signals or measured impedance.

In an embodiment, a pacing event may be selectively controlled and adjusted by a pacing event activator means. Pacing event activator may be a input sensor such as a push button, depressed for a predefined period of time. In an embodiment, pulse generator 36 comprises capacitors that are successively charged and discharged.

In an embodiment, pulse generator 42 comprises a switch-mode power-supply.

In an embodiment, distal portion 18 of elongated member 14 may include one or more pacing electrodes 38. In an implementation, pulse generator 36 paces between the reference electrode 20 and the one or more pacing electrodes 38. In an embodiment, pulse generator 36 paces between the reference electrode and the one or more pacing electrodes through a single cable insert extending from the handle 12.

In an embodiment, pulse generator 36 may further include protective circuitry to protect pulse generator 36 from generating harmful pulses. In an implementation, the protection circuitry comprises a input sensor 90 and a timer. In another embodiment the protection circuit is controlled by microcontroller 32 and may communicate with a remote control system or other catheters.

In an embodiment, any of the members of the handle 12, such as input sensor 90 and/or output generator 92, are protected from defibrillator and ablation currents. Among other possibilities, protection from defibrillation and ablation may comprise one or more diodes in series with one or more resistors. In another embodiment protection from defibrillators and ablation currents is achieved from a high input impedance of the system.

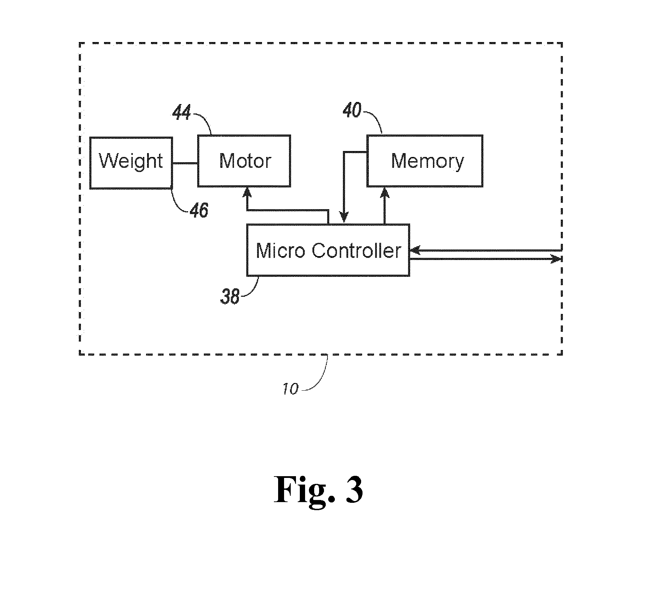

FIG. 3 illustrates an embodiment of a catheter, wherein the catheter comprises a motor 44. In an embodiment the rotation of motor 44 is controlled by microcontroller 38 by pulse-width-modulation (PWM). In an embodiment, the motor 44 may include a weight 46 that is shaped as a semicircle to displace the center of weight with respect to an axis of rotation. In the aforementioned embodiment, rotation of the weight causes vibration. In an embodiment, the motor comprises a permanent magnet DC motor and gearing.

In an embodiment, a pressure sensor is disposed upon distal portion 18 and the motor 44 provides a scaled vibration response. In an embodiment, the scaled vibration response is a function of the measurements experienced by pressure sensor 24. In an embodiment, the scaled vibration response is a function of cardiac signals. In an embodiment, the scaled vibration response is a function of other external inputs. External inputs may be commands digitally communicated to the microcontroller 38. One embodiment is the vibration response to the confirmation of a pacing event.

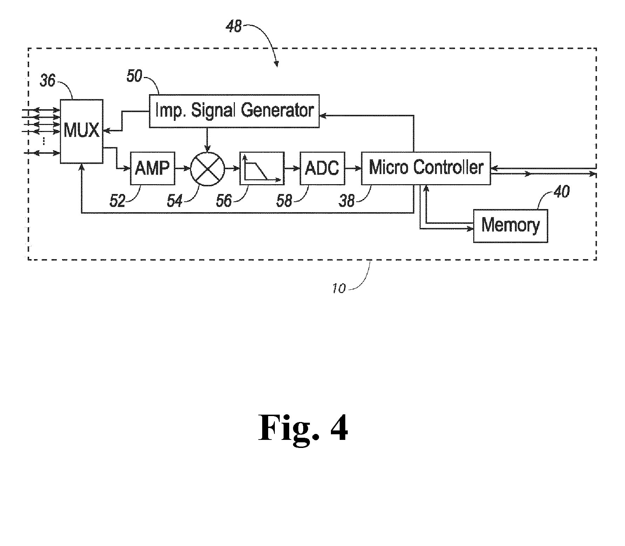

FIG. 4 illustrates an embodiment of a catheter, wherein the catheter comprises an impedance measurement device 48 that includes an impedance signal generator 50. Impedance signal generator 50 induces a high frequency AC signal between two or more electrodes at a selected frequency range. In an embodiment, the selected frequency range may be higher than 10kHz. A voltage may be simultaneously measured between the electrodes or another pair of electrodes. The measured signal passes through an amplifier 52. The induced signal from the impedance signal generator 48 may then be passed to a demodulator 54, along with the amplified signal, to obtain a measured response. Thereafter, the measured response is transmitted to a low-pass filter 56 whereupon it is filtered before it is digitized by an analog-to-digital converter 58 and sent to microcontroller 38. The digital values are translated into a measure of impedance.

In an embodiment, the microcontroller 38 may control steering the catheter as a function of impedance measurements. In an embodiment, catheter 10 includes an algorithm for motor control of the elongated member. In one embodiment, motor control is automatically controlled as a function of impedance measurements. In an embodiment, motor control is controlled as a function of pressure sensor measurements.

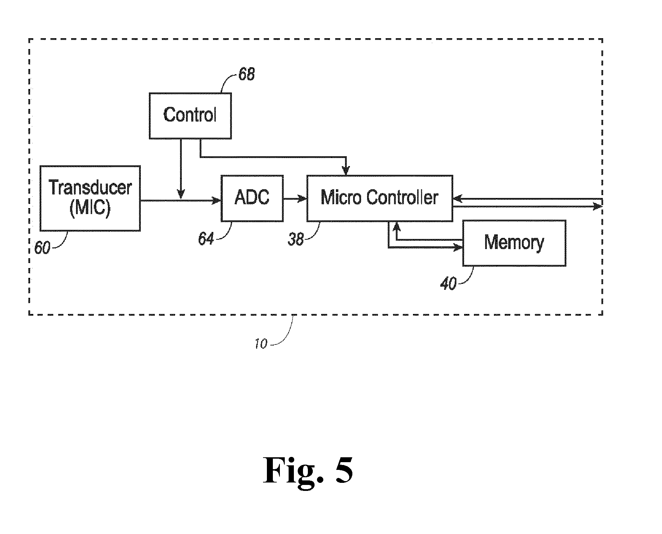

FIG. 5 illustrates an embodiment of a catheter, wherein the input sensor 90 comprises circuitry for voice recognition. In an embodiment, input sensor 90 includes a transducer 60, an analog-to-digital converter 64, microcontroller 38 and memory module 40.

In an embodiment, transducer 60 converts acoustic waves to electric signals. The signals are passed through the analog-to-digital converter 64 to the microcontroller 38 where it may be processed. Memory 40 may be configured to facilitate two-way communication with microcontroller 38 and utilized for processing the recorded signals. In an embodiment, the transducer 60 is installed in vicinity of the patient bed.. In an embodiment, transducer 60 is integrated in the handle 12. In another embodiment, the transducer 60 is enabled by a input sensor 90.

As described, function means may employ voice recognition members. In such an embodiment voice recognition is employed to control pacing. For example, a physician initiates the pacing control by a predefined verbal command such as 'pace'. As may be appreciated, the first parameter to set is pulse amplitude. The accepted pulse amplitude range is predefined such as from 0 to 20 milli amperes. Second, the physician commands the pulse width. Normal ranges are 0.5 to 2ms. Third, the physician commands interval length between pulses. The minimal accepted value is pre-set such as 200ms. Lastly, the physician commands which pole to pace through. An example is '4' and may vary from system to system. Settings related to pulse amplitude, pulse width and repetition may be pre-set and a physician only needs to state 'start pacing'. A confirmation message of the pacing setting is provided. In one embodiment, the physician confirms the pacing by 'Yes'. In an embodiment, the physician or staff confirms the pacing by a button on the catheter handle. In another embodiment pacing parameters are adjusted and confirmed by a combination of voice and button interactions. It is to be appreciated that the voice recognition may be used to control other electronic catheter functions and the invention should not be so limited to the disclosed examples.

For example, in an implementation, the voice recognition feature may be employed to measure electrograms. In one embodiment, the physician initiates the function by a command such as 'freeze'. This command will freeze the monitor's streaming. This may at anytime be cancelled by the command 'stream'. The command 'measure' enables at least one function 'interval'. The physician commands which channel to look at. An example is 'HIS'. The physician commands which part of the x-axis to look at.

As another example, the voice recognition feature may be employed to control steering. The physician activates this function by commanding 'steer'. The physician commands in which direction the catheter tip to move laterally. The physician may command 'obtain tissue contact'. In one embodiment, there are two options, either 'bend' or 'extend'. The physician commands the distance for the tip to move. In one embodiment with a pressure sensor on the catheter's tip, voice controlled steering gives a warning about increasing tissue contact in a moving direction and cancels steering over a threshold value.

In yet another example, the voice recognition feature may be employed to control monitor display. In one embodiment, the physician commands which leads to be depicted on the monitor. An example is 'HIS' or 'RV'.

An embodiment of integrated circuitry for voice recognition is impedance measurements by first stating which pole, example '3', followed by '4' or 'to ground' to indicate whether the reference electrode for the measurement is a band electrode or a distant reference voltage.

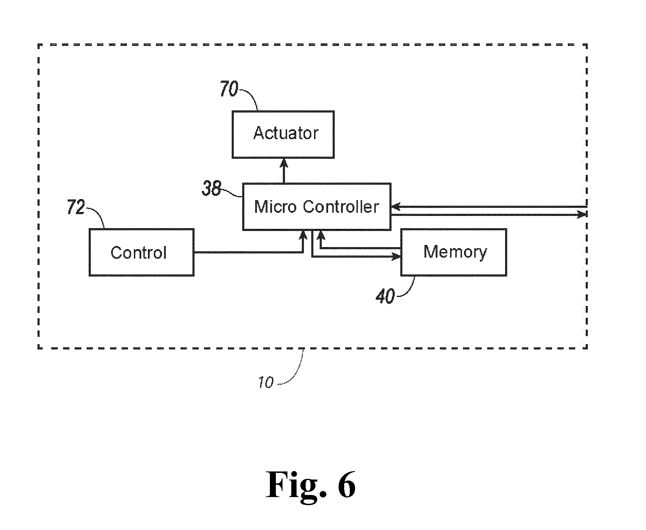

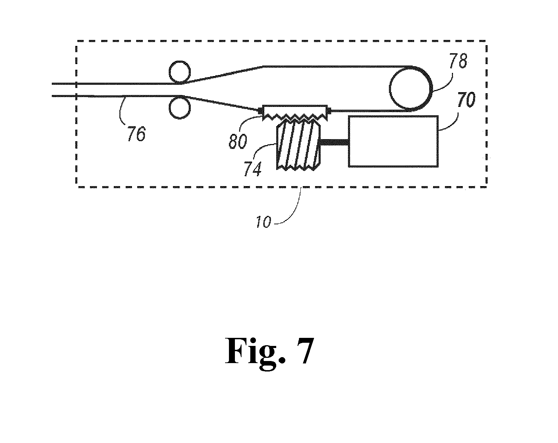

FIG. 6 and 7 illustrate implementations of catheters, wherein the handle comprises an actuator 70 in drivingly connected to a rotary 74. In an embodiment, a metal wire 76 extends around pulley 78 and is connected to a position element 80. Position element 80 in conjunction with rotary 74 effects linear movement of the wire 76 in response to rotary movement of the actuator 70. In an embodiment the wire is connected to the distal portion 18 of elongated member 14. Movement of the wire enables control of curvature of the distal portion 18. In an embodiment microcontroller 38 communicates with both input sensor 90 and actuator 70. input sensor 90 controls the initiation method for actuator activity and interfaces with other integrated functionality, such as those described herein. In an embodiment, input sensor 90 interfaces with a hardware interface unit on a button mechanism on the handle. In another embodiment, input sensor 90 interfaces with voice control circuitry. FIG. 7 illustrates actuator 70 and rotary 74 mechanically connected to allow a rotation motion of the actuator 70 to pull or release the ends of the metal wire via rotary 74. In one embodiment, the actuator 70 is a stepper motor.

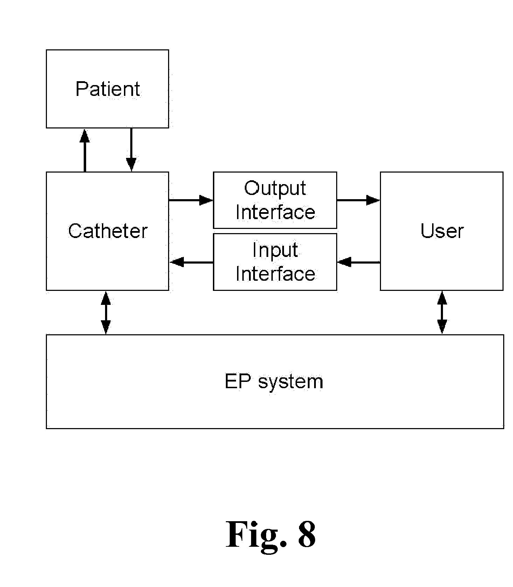

FIG. 8 illustrates an embodiment of a system comprising a catheter in relation to a patient; the catheter interacting with a user through input sensor and output generator; an EP system communicating with the catheter and the user. The catheter provides input signals to the EP system in response to EP signals being present at the catheter. In one embodiment the EP system comprises at least one or more monitors. In such an embodiment the EP system may provide information to the user through the monitors.

With continued reference to FIG. 8, in one embodiment the user communicates with the EP system via the catheter through the input sensor. The input sensor may comprise methods for physical input such as pushbuttons, touch sensing or proximity sensing as well as voice sensing. In such an embodiment the input sensor may enable the user to configure the EP system through the catheter. In another embodiment the EP system communicates with the user via the catheter through the output generator. In such an embodiment communication to the user through the output generator may be effected by a display, sound, light or vibration. An example of such communication may be vibration in response to changes in configuration of the EP system.

With continued reference to FIG. 8, in one embodiment, the user interacts with the patient via the catheter through the input sensor and the output generator. Examples of such interaction may be vibration in response to a biophysical sensor measuring signals beyond a predefined or adaptive threshold or pacing controlled by the user through the user input sensor.

With continued reference to FIG. 8, in one embodiment, the EP system enables interaction between the user and the patient. In such an embodiment, examples of interaction are pacing or biophysical measurements.

With continued reference to FIG. 8, in an embodiment, communication between members is redundant. In an embodiment communication may be used to verify functionality of members of the system. In an embodiment, interaction between members is interdependent. In such an embodiment interaction may be pacing of the patient through the catheter; pacing initiated by the user in communication with the EP system; the pacing confirmed to the user by the EP system through one or more monitors; pacing effected through the catheter; the catheter configured by the user through the user input sensor, the configuration confirmed to the user by the catheter through the output generator.

With continued reference to FIG. 8, in an embodiment configuration of the catheter and the EP system is performed by connecting the catheter to the EP system or wirelessly pairing the catheter and the EP system. In one embodiment, configuration comprises automatic procedures that uses information about catheter specifications. In an embodiment, the catheter provides information to the electrophysiology system about past events related to the catheter. Examples of such information may be the number and type of biophysical sensors, the catheter production, use history, the mechanical properties of the catheter and information that may be used for calibration of catheter sensors.

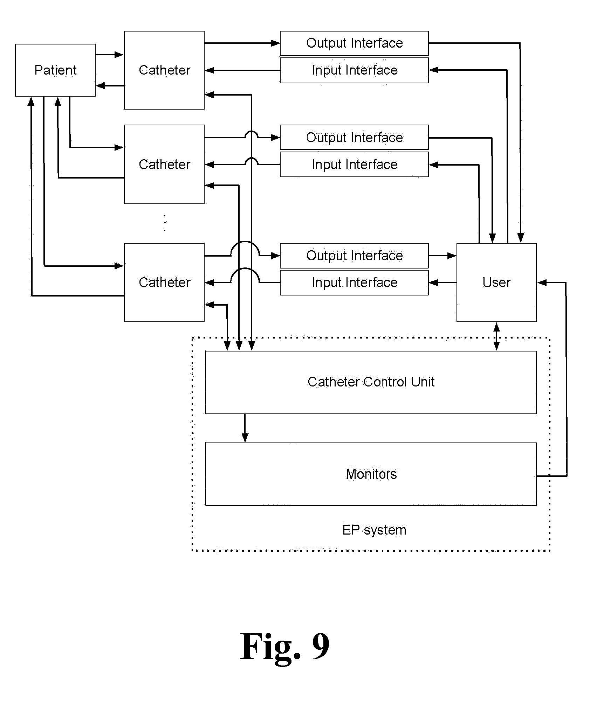

FIG. 9 illustrates an embodiment of a system comprising 3 catheters in relation to a patient, the catheters interacting with a user through one user input sensors and one output generator per catheter; the catheters communicating with an EP system comprising a set of monitors, and a catheter control unit; the monitors displaying information to the user; the catheter control unit communicating with the user. In one embodiment the user configures the EP system through the user input sensor of one or more catheters. In one embodiment the user configures the one or more catheters through the catheter control unit.

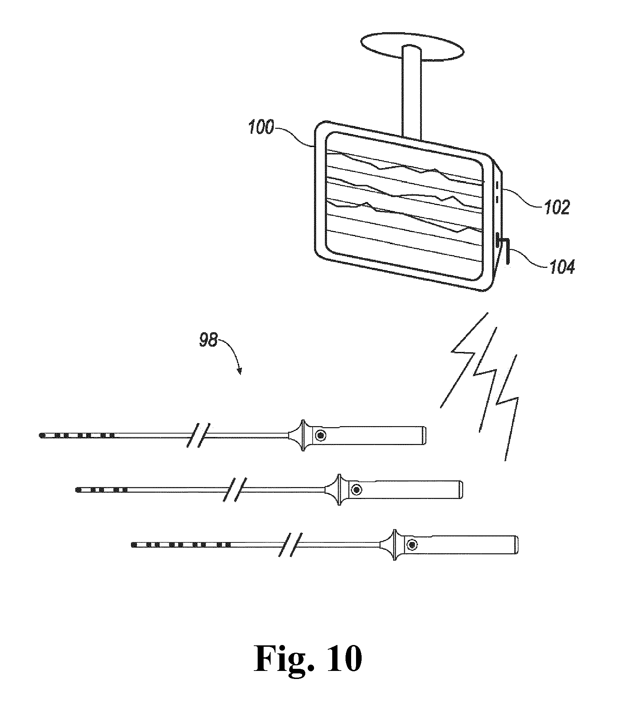

FIG. 10 illustrates an embodiment of a system, comprising 3 catheters 98 and 1 monitor 100 with data processing module 102 and antenna 104 for RF communication. In an implementation, one or more catheters 98 and one or more monitors 100 have a direct digital link. In an embodiment, the direct digital link is wireless. It is obvious to a reasonable skilled person in the art that wireless communication may substitute wires in any part of the system. In an implementation, one or more monitors control functions of the one or more catheters. In a particular implementation, the one or more monitors comprise memory and processing power to react to critical values by informing the catheter user. Informing the catheter user may be through vibrating the handle. Critical values may be for impedance, pressure or electrogram peak-to-peak. In another implementation, the user input sensor 90 on the catheter handle 12 enable controlling the monitor. Controlling the monitor may be moving a curser to adjust settings or confirm warnings. In an embodiment, one or more transceivers enable voice control as a means for functioning the one or more monitors.

In an embodiment, the monitor and catheter comprises the needed instrumentation to conduct electrophysiology measurements. The physician measures and paces with no need for a separate work station. In an embodiment, the one or more catheters and one or more monitors comprise a system with automatic detection and channel pairing. The one or more catheters are steered with leadless handles.

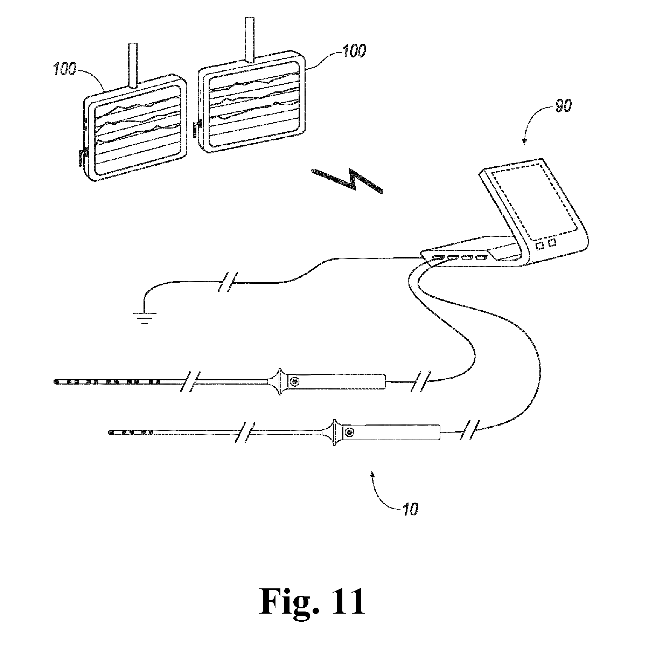

FIG. 11 illustrates an embodiment of a system, wherein a catheter interface unit 90 (CIU) communicating with 2 catheters also communicates with 2 monitors. In an implementation, the CIU provides power to the one or more catheters and may provide electrical ground reference for the catheters. In one embodiment, the CIU includes connectivity to the Internet. In one implementation, communication with the one or more catheters is wireless. In an implementation, the CIU comprises means for voice control.

In one embodiment, the CIU provides means for functioning of the catheters such as impedance measurements and pace generation. The CIU may include isolated power supply and control circuitry for safely delivering stimulation pulses through electrodes of the catheters.

In one embodiment, the CIU includes a display and a user input sensor. In one implementation the physical interface is a touch sensitive screen. The display may monitor physiological signals as well as displaying other information to the user such as information originating from the catheters or other members of the system. The user input sensor may be used for controlling functions of other members of the system such as catheters or monitors. The user input sensor may be used for adjusting parameters for functions of the CIU. The user input sensor may be used in connection with user input sensors of other members of the system.

In an embodiment, communication interface 88 comprises a cable insertion point that may facilitate power transmission, input and output signals, charging a rechargeable power source that may be integrated in the handle, providing a ground connection, and the like.

In one embodiment, the CIU comprises a digital-to-analog (DAC) converter and an output generator. The DAC may be used to produce an analog output correlating with signals originating from the catheters. The output generator may be utilized to achieve compatibility with other electrophysiology systems.

## Claims

1. A catheter assembly comprising:
• an elongated member having a proximal portion and a distal portion, the distal portion being configured to be inserted into a mammal body;
• a plurality of electrodes provided at the distal portion of the elongated member, the plurality of electrodes being configured to provide one or more input signals in response to electrophysiology signals being present at the electrodes;
• a handle connected to the proximal portion of the elongated member, the handle enabling a user of the catheter assembly to maneuver the elongated member during insertion of the distal portion within a human body;
• a user interaction system provided at the handle, the user interaction system comprising at least one user input sensor enabling the user to provide input to the catheter assembly, the user interaction system comprising at least one output generator for providing output to the user.

2. The catheter assembly according to claim 1, wherein the at least one user input sensor comprises a microphone facilitating voice recognition.

3. The catheter assembly according to any of the preceding claims, wherein the at least one user input sensor comprises a finger controllable part, such as a touch and/or force sensing element, such as a push button and/or a touch panel.

4. The catheter assembly according to any of the preceding claims, wherein the at least one user output generator comprises a vibration generating element.

5. The catheter assembly according to any of the preceding claims, wherein the at least one user output generator comprises a display.

6. The catheter assembly according to any of the preceding claims, wherein the at least one user output generator comprises a speaker.

7. The catheter assembly according to any of the preceding claims, wherein the catheter assembly is configured for providing output to the user in response to the input signals.

8. The catheter assembly according to any of the preceding claims, wherein at least the distal portion of the elongated member is bendable and wherein the catheter assembly comprises means for controlling the bend of the distal portion.

9. A system for electrophysiology measurements within a mammal body, the system comprising the catheter assembly according to any of the preceding claims and an electrophysiology system associated with the catheter.

10. The system according to any of the preceding claims, wherein the electrophysiology system comprises catheter control unit comprising a pulse generator configured for pacing of heart tissue via one or more of the plurality of electrodes.

11. The system according to claim 9 or 10, wherein at least one of the at least one user input sensor is configured for controlling the electrophysiology system.

12. The system according to any of the claims 9-11, wherein the electrophysiology system is configured to provide output to the user via at least one of the at least one user output generator, the output from the electrophysiology system for instance being provided in response to input sensed via the at least one user input sensor or via at least one of the plurality of electrodes.

13. The system according to any of the claims 9-12, wherein the catheter assembly is configured for providing information to the electrophysiology system about past events related to the catheter.

14. A method for electrophysiology measurements within a mammal body, the method comprising utilizing the catheter assembly according to any of the claims 1-8 or the system according to any of the claims 9-13 for obtaining electrophysiology measurements.
